(19) Europäisches Patentamt
European Patent Office
Office européen des brevets

(11) **EP 4 786 974 A1**

(12) **EUROPEAN PATENT APPLICATION**
published in accordance with Art. 153(4) EPC

(43) Date of publication:
**05.08.2026 Bulletin 2026/32**

(21) Application number: **24872197.9**

(22) Date of filing: **24.09.2024**

(51) International Patent Classification (IPC):
**G01N 33/2028** (2019.01)    **C22C 38/00** (2006.01)
**C22C 38/60** (2006.01)    **G06N 3/0455** (2023.01)
**G06N 20/00** (2019.01)

(52) Cooperative Patent Classification (CPC):
**C22C 38/00; C22C 38/60; G01N 33/2028;
G06N 3/0455; G06N 20/00**

(86) International application number:
**PCT/JP2024/033968**

(87) International publication number:
**WO 2025/070407 (03.04.2025 Gazette 2025/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.09.2023 JP 2023163687**

(71) Applicant: **Proterial, Ltd.**
**Tokyo 135-0061 (JP)**

(72) Inventor: **FUKUMOTO, Shiho**
**Tokyo 135-0061 (JP)**

(74) Representative: **Cabinet Beaumont
4, Place Robert Schuman
B.P. 1529
38025 Grenoble Cedex 1 (FR)**

(54) **STEEL EVALUATION METHOD, PROGRAM, AND MODEL GENERATION METHOD**

(57)    Provided is a method for evaluating steel and the like capable of evaluating steel satisfying standards related to composition in more detail.

The method for evaluating steel includes analyzing a composition of steel, when composition data obtained by analysis satisfies a predetermined standard, inputting the composition data to a model trained by machine learning to output the same data as input data and calculating an error loss between output data from the model and the composition data, and determining whether or not the error loss exceeds a predetermined reference value.

FIG.1

EP 4 786 974 A1

**Description**

Technical Field

The present invention relates to a method for evaluating steel, program, and method for generating a model.

Background Art

Steel is an alloy obtained by adding small amounts of additives such as carbon, silicon, manganese, and phosphorus to iron. Since properties of steel greatly vary depending on the ratios of the respective components, that is, the composition, for example, various types of steel such as carbon steel, tool steel, and spring steel are manufactured and used. For each type of steel, standards related to composition are defined (Patent Literature 1).

Citation List

Prior Art Documents

Patent Literature:

Patent Literature 1: Japanese Patent Application Laid-Open Publication No. 2006-152356

Summary of the Invention

Problems to be Solved by the Invention

In the above-described standards, the ratios of the respective components are specified as ranges. When manufacturing steel, content of each component are adjusted to fall within the defined ranges, that is, to satisfy the standards related to the components. For the manufactured steel, various shipment inspections including composition analysis for confirming that the standards related to composition are actually satisfied are performed. Steel passing the shipment inspections is shipped.
However, when a plurality of lots of steel manufactured to satisfy the same standards related to composition are compared with each other, it is empirically known that there may be steel that exhibits superior properties compared with steel of other lots. By detecting occurrence of such superior properties of steel and reflecting the detection in management of a manufacturing process, an overall improvement in steel quality can be expected.
In one aspect, an object is to provide a steel evaluation method and the like capable of evaluating steel satisfying standards related to composition in more detail. Means for Solving the Problem
A method for evaluating steel, the method comprising: analyzing a composition of steel; when composition data obtained by analysis satisfies a predetermined standard, inputting the composition data to a model trained by machine learning to output the same data as input data, and calculating an error loss between output data from the model and the composition data; and determining whether or not the error loss exceeds a predetermined reference value. Advantageous Effects of the Invention
In one aspect, it is possible to provide a steel evaluation method and the like capable of evaluating steel satisfying standards related to composition in more detail.

Brief Description of the Drawings

FIG. 1 is an explanatory diagram illustrating a process of determining and using a steel evaluation method.
FIG. 2 is an explanatory diagram illustrating a utilization stage of the steel evaluation method.
FIG. 3 is a table illustrating compositions of samples used in a preliminary study stage.
FIG. 4 is a graph illustrating heat-treatment-induced dimensional-change rates of steel having different molybdenum contents arranged side by side.
FIG. 5 is a graph illustrating heat-treatment-induced dimensional-change rates of steel having different chromium contents arranged side by side.
FIG. 6 is a graph illustrating heat-treatment-induced dimensional-change rates of steel having different vanadium contents arranged side by side.
FIG. 7 is a graph illustrating heat-treatment-induced dimensional-change rates of steel having different silicon contents arranged side by side.

FIG. 8 is a graph illustrating heat-treatment-induced dimensional-change rates of steel having different manganese contents arranged side by side.

FIG. 9 is an explanatory diagram illustrating a configuration of an information processing apparatus used to generate a model.

FIG. 10 is an explanatory diagram illustrating the model.

FIG. 11 is a flowchart illustrating a processing flow of a program for generating the model.

FIG. 12 is a graph illustrating variation in mean absolute error loss according to manufacturing time.

FIG. 13 is an explanatory diagram illustrating a configuration of a steel evaluation system.

FIG. 14 is a flowchart illustrating a processing flow of the program in a utilization stage.

Description of the Invention

[Embodiment 1]

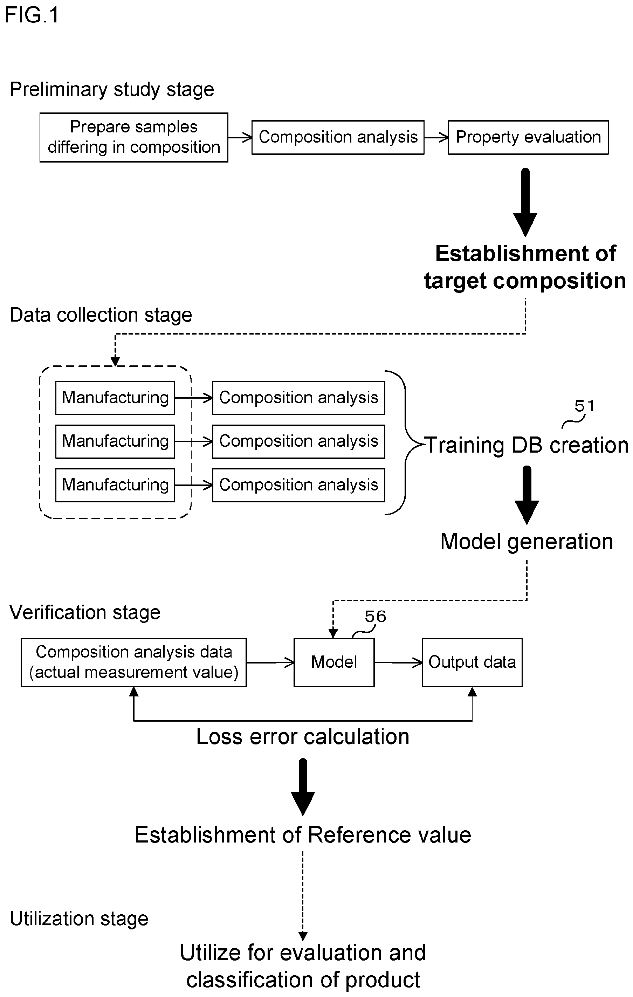

FIG. 1 is an explanatory diagram illustrating a process of determining and using a steel evaluation method. In the present embodiment, a case is described as an example in which, for steel that is already being mass-produced, the composition is finely adjusted to stably reproduce excellent properties, and an evaluation method for stably manufacturing steel after the fine adjustment is determined. An outline of a process of determining the steel evaluation method will be described with reference to FIG. 1.

First, a preliminary study stage is performed. In the preliminary study stage, a plurality of steel samples having slightly different compositions are prepared. The samples include steel having standard properties from among steel being mass-produced, and steel in which the content of each component is intentionally increased or decreased.

For each sample, composition analysis is performed. In addition, property evaluation is performed for each sample. Based on the results of the composition analysis and the property evaluation, a target composition for stably manufacturing steel having desirable properties is established.

Here, the target composition is defined within the specified range for the content of each component defined in the standard of the mass-produced steel, so as to set the ratio of specific components to be relatively higher or lower. In other words, the target composition does not deviate from the standard of the mass-produced steel. Details of the preliminary study stage will be described later.

Next, a data collection stage is carried out. In the data collection stage, a plurality of lots of steel are manufactured under manufacturing conditions modified based on the target composition established in the preliminary study stage. Note that the components of the steel actually manufactured have vary among lots.

For each lot of steel, composition analysis is performed. A training DB (Database) 51 in which a plurality of sets of composition analysis data is recorded is created. Using the training DB 51, a model 56 is generated. The model 56 is, for example, an autoencoder, and is trained by machine learning to restore and output the same data as the input composition analysis data. Details of the model 56 will be described later.

Next, a verification stage is carried out. In the verification stage, composition analysis data measured for a plurality of lots of steel manufactured prior to the preliminary study stage, and composition analysis data measured for a plurality of lots of steel manufactured in the data collection stage, are used. Each set of composition analysis data is input to the model 56, and output data are obtained from the model 56. An error loss between the input composition analysis data and the output data is calculated.

In the present embodiment, the error loss is defined as a LOSS MAE (mean absolute error). When the composition of the input data is similar to the training data used to generate the model 56, the error loss becomes small. Conversely, when the difference between the composition of the input data and the training data is large, the error loss becomes large.

Accordingly, the error loss calculated for steel manufactured prior to the data collection stage tends to be larger than the error loss calculated for steel manufactured in the data collection stage. A reference value of the error loss is established at a level that allows distinction between a variation range of steel lots manufactured after the data collection stage and a variation range of steel lots manufactured before the data collection stage. Details of the verification stage will be described later.

Finally, an outline of a utilization stage will be described. For each lot of manufactured steel, an error loss is calculated in the same manner as in the verification stage. Based on the established reference value, evaluation of the manufactured steel and the like is performed. Details of the utilization stage will be described later.

An outline of the significance of establishing the reference value of the error loss will be described. Steel having a composition similar to the target composition established in the preliminary study stage is expected to exhibit favorable properties. However, since the content of each component varies due to manufacturing variation, it is difficult for a manufacturing technology engineer to determine whether the properties are similar or different between the measured composition and the target composition by viewing the composition data.

By using the reference value of the error loss established in the verification stage, similarity or difference from the target composition can be easily determined. That is, when the calculated error loss is equal to or less than the reference value, the composition of the manufactured steel is similar to that of the steel manufactured in the data collection stage, and it can be determined that the steel has desirable properties. When the calculated error loss exceeds the reference value, the composition of the manufactured steel differs from that of the steel manufactured in the data collection stage, even when a predetermined standard is satisfied, and it can be determined that the steel does not have particularly desirable properties.

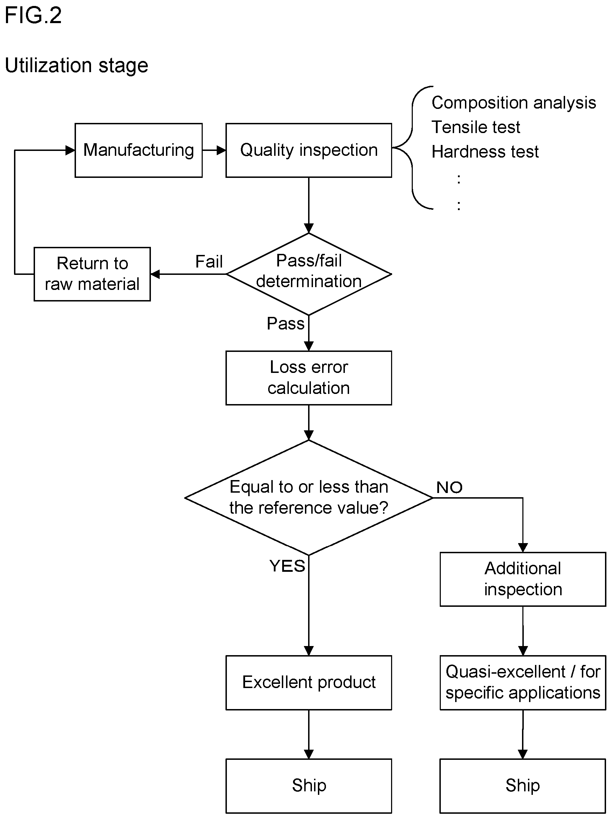

FIG. 2 is an explanatory diagram illustrating the utilization stage of the steel evaluation method. For newly manufactured steel, various quality inspections including composition analysis are performed. Based on the results of the quality inspections, a conformity determination is made as to whether the steel satisfies a predetermined standard. Steel determined to be nonconforming is not shipped and is instead used as a raw material for manufacturing new steel. Since the conformity determination in steel quality inspection has been conventionally carried out, detailed description thereof will be omitted.

For steel determined to be conforming (conforming product), the composition analysis data is input to the model 56. An error loss between the output data from the model 56 and the composition analysis data is calculated. When the error loss is equal to or less than the reference value, the steel is determined to be an excellent product having desirable properties. For example, excellent products are delivered to customers that require particularly high reliability.

When the error loss exceeds the reference value, the steel is a conforming product that satisfies a predetermined standard, but is determined not to be an excellent product. In the following description, a product that is a conforming product but not an excellent product is referred to as a quasi-excellent product. For quasi-excellent products, for example, additional inspections are performed in consideration of applications and the like used at delivery destinations. Based on the results of the additional inspections, quasi-excellent steel products are classified into ordinary conforming products and conforming products for specific applications and the like, and are shipped to appropriate customers.

As described above, steel determined to be a conforming product by conventional quality inspection is classified into excellent products and quasi-excellent products based on the reference value, and the quasi-excellent products are further classified according to customer applications by additional inspection, thereby enabling each steel product to be shipped to an appropriate customer according to their properties.

[Preliminary study stage]

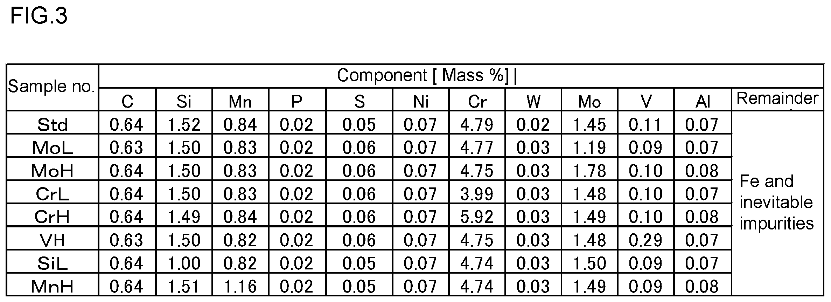

FIG. 3 is a table illustrating compositions of samples used in the preliminary study stage. Eight types of samples are used. Each sample is distinguished by the sample number shown at the left end of FIG. 3. For each sample, each component is expressed in mass percent.

"Std" indicates steel having standard properties among mass-produced steel. "MoL" indicates steel having a molybdenum (Mo) content smaller than that of "Std". "MoH" indicates steel having a molybdenum content larger than that of "Std". "CrL" indicates steel having a chromium (Cr) content smaller than that of "Std". "CrH" indicates steel having a chromium content larger than that of "Std". "VH" indicates steel having a vanadium (V) content larger than that of "Std". "SiL" indicates steel having a silicon (Si) content smaller than that of "Std". "MnH" indicates steel having a manganese (Mn) content larger than that of "Std".

For each of the samples illustrated in FIG. 3, six rectangular-parallelepiped specimens were prepared. Each specimen was cut from a single steel material such that a direction in which the steel material was elongated by hot working is defined as a length direction, a direction in which the steel material was compressed is defined as a thickness direction, and a direction orthogonal to both the length direction and the thickness direction is defined as a width direction. In the graphs used in the following description, the length direction is indicated by symbol L, the width direction is indicated by symbol W, and the thickness direction is indicated by symbol T.

For each specimen, a heat-treatment-induced dimensional-change rate was measured before and after heat treatment. The heat-treatment-induced dimensional-change rate indicates a dimensional-change rate calculated by dividing the amount of dimensional change due to heat treatment by the dimension before heat treatment. Measurement of the heat-treatment-induced dimensional-change rate is an example of a property evaluation method in the preliminary study stage described using FIG. 1.

The heat treatment includes quenching and tempering. The quenching conditions are all identical. Quenching treatment was performed by semi-cooling from 1020°C for 10 minutes. The value of semi-cooling indicates the time required to cool to half of 1020°C (510°C). Tempering was performed under five conditions of tempering temperatures of 485°C, 500°C, 515°C, 530°C, and 545°C. At each temperature, tempering treatment was performed twice.

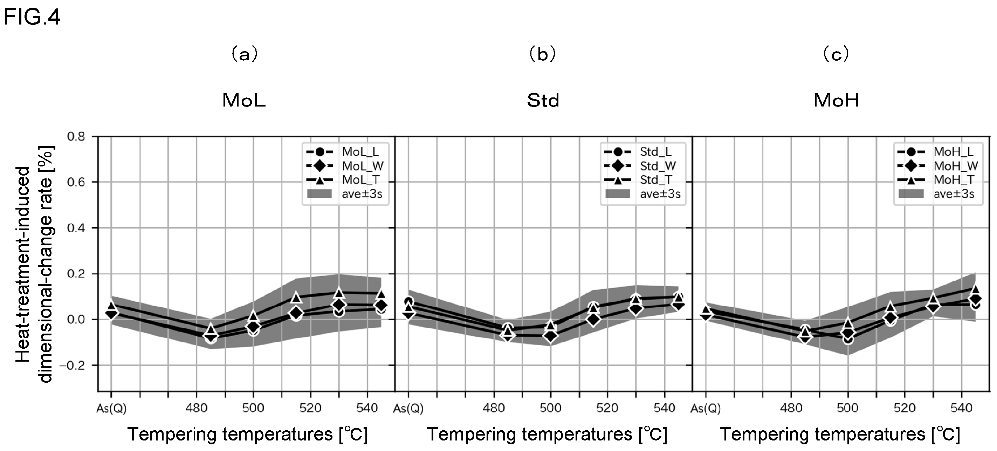

FIG. 4 is a graph illustrating heat-treatment-induced dimensional-change rates of steel having different molybdenum contents arranged side by side. In FIG. 4, three graphs sharing a common vertical axis are horizontally arranged. The leftmost part (a) illustrates a heat-treatment-induced dimensional-change rate of "MoL", the central part (b) illustrates a heat-treatment-induced dimensional-change rate of "Std", and the rightmost part (c) illustrates a heat-treatment-induced dimensional-change rate of "MoH", respectively.

A horizontal axis of the graphs indicates the tempering temperature, and a vertical axis indicates the heat-treatment-induced dimensional-change rate. The heat-treatment-induced dimensional-change rate on the vertical axis is a value calculated by measuring the sample dimension before heat treatment and the sample dimension after heat treatment by dividing the dimensional change before and after heat treatment by the dimension before heat treatment. A positive value on the vertical axis indicates expansion due to heat treatment, and a negative value indicates contraction due to heat treatment. "As (Q)" on the horizontal axis indicates a sample subjected only to quenching.

A black circle in the graph indicates the heat-treatment-induced dimensional-change rate in the length direction (L). A black diamond indicates the heat-treatment-induced dimensional-change rate in the width direction (W). A black triangle indicates the heat-treatment-induced dimensional-change rate in the thickness direction (T). For each temperature, the average value ave and the sample standard deviation s of the three heat-treatment-induced dimensional-change rates in the length direction, the width direction, and the thickness direction are calculated, and a range of ave ± 3s is illustrated in gray.

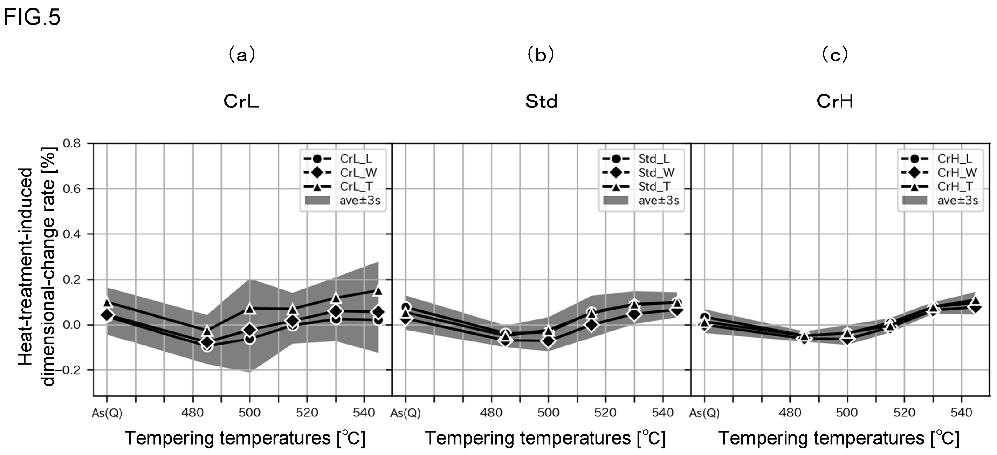

FIG. 5 is a graph illustrating heat-treatment-induced dimensional-change rates of steel having different chromium contents arranged side by side. In FIG. 5, three graphs sharing a common vertical axis are horizontally arranged. A leftmost part (a) illustrates a heat-treatment-induced dimensional-change rate of "CrL", a central part (b) illustrates a heat-treatment-induced dimensional-change rate of "Std", and a rightmost part (c) illustrates a heat-treatment-induced dimensional-change rate of "CrH", respectively. Since the vertical axis, the horizontal axis, and plot symbols are the same as those in FIG. 4, description thereof is omitted. FIG. 4(b) and FIG. 5(b) are the same graph.

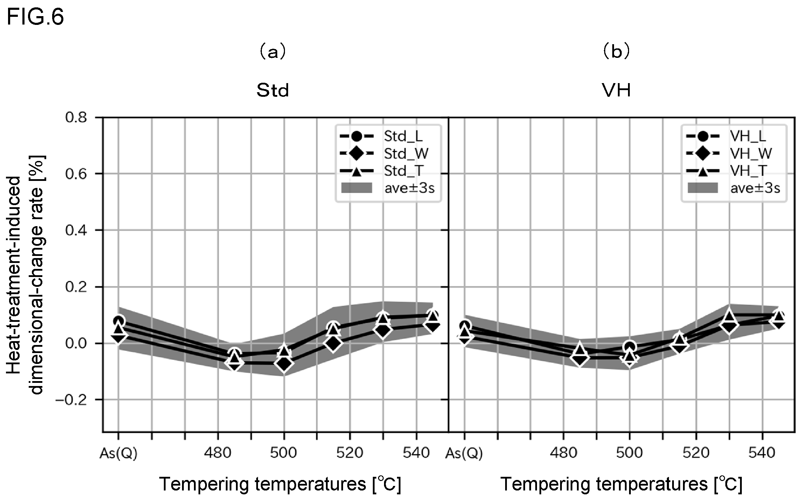

FIG. 6 is a graph illustrating heat-treatment-induced dimensional-change rates of steel having different vanadium contents arranged side by side. In FIG. 6, two graphs sharing a common vertical axis are horizontally arranged. A left part (a) illustrates a heat-treatment-induced dimensional-change rate of "Std", and a right part (b) illustrates a heat-treatment-induced dimensional-change rate of "VH", respectively. Since the vertical axis, the horizontal axis, and plot symbols are the same as those in FIG. 4, description thereof is omitted. FIG. 4(b) and FIG. 6(a) are the same graph.

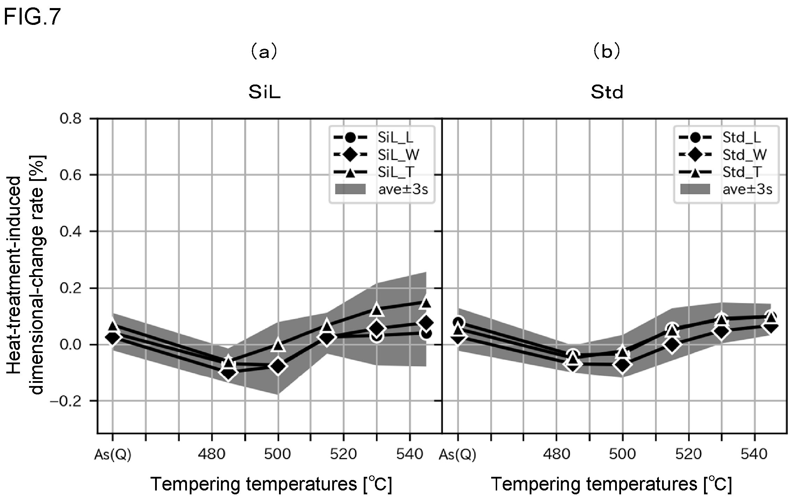

FIG. 7 is a graph illustrating heat-treatment-induced dimensional-change rates of steel having different silicon contents arranged side by side. In FIG. 7, two graphs sharing a common vertical axis are horizontally arranged. A left part (a) illustrates a heat-treatment-induced dimensional-change rate of "SiL", and a right part (b) illustrates a heat-treatment-induced dimensional-change rate of "Std", respectively. Since the vertical axis, the horizontal axis, and plot symbols are the same as those in FIG. 4, description thereof is omitted. FIG. 4(b) and FIG. 7(b) are the same graph.

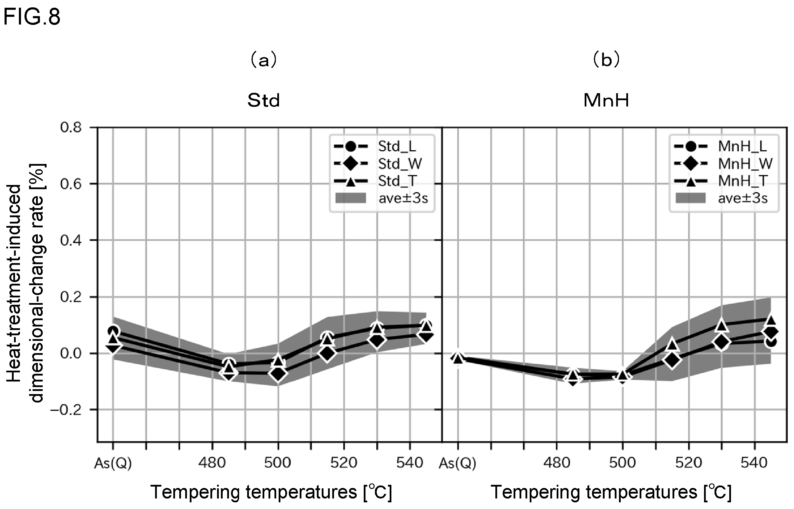

FIG. 8 is a graph illustrating heat-treatment-induced dimensional-change rates of steel having different manganese contents arranged side by side. In FIG. 8, two graphs sharing a common vertical axis are horizontally arranged. A left part (a) illustrates a heat-treatment-induced dimensional-change rate of "Std", and a right part (b) illustrates a heat-treatment-induced dimensional-change rate of "MnH", respectively. Since the vertical axis, the horizontal axis, and plot symbols are the same as those in FIG. 4, description thereof is omitted. FIG. 4(b) and FIG. 8(a) are the same graph.

When steel having large anisotropy in heat-treatment-induced dimensional-change rate is machined, differences in shape due to dimensional change occur as a result of heat treatment after machining, thereby increasing machining costs for shape correction or increasing the number of machining processes. For this reason, as a property of steel, it is desirable that anisotropy of the heat-treatment-induced dimensional-change rate among steel be small, that is, that the width of the range of ave ± 3s illustrated in gray in the graphs of FIGS. 4 to 8 be narrow.

Furthermore, since heat-treatment temperatures such as tempering and the like are appropriately selected depending on the equipment used and the mechanical properties of the manufactured machine parts, it is difficult to take heat-treatment-induced dimensional-change into consideration. Therefore, as a property of steel, it is desirable that differences in heat-treatment-induced dimensional-change rate due to tempering temperature be small, that is, that changes in the vertical-axis direction in the graphs of FIGS. 4 to 8 be small.

In view of the above criteria, it is understood that the steel having higher chromium content illustrated in FIG. 5(c) and the steel having higher vanadium content illustrated in FIG. 6
(b) exhibit excellent properties with small variations in heat-treatment-induced dimensional-change rate depending on the direction and temperature. Therefore, in the preliminary study stage, a target composition in which the chromium and vanadium contents are increased is established.

[Data collection stage]

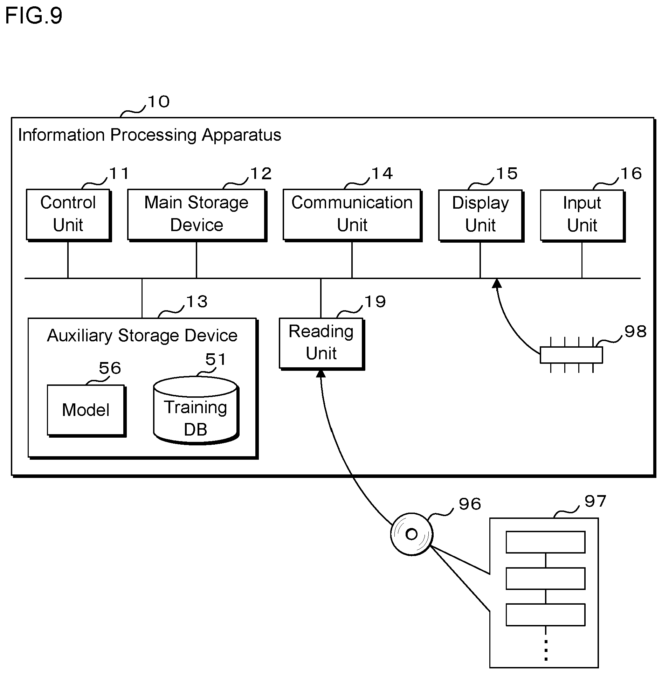

Based on the target composition, 18 lots of steel in which chromium and vanadium contents are increased within a standard range are manufactured (after the target composition is established). For each lot of steel, composition analysis is performed, and the training DB 51 in which composition analysis data are recorded is created (FIG. 9 to be described later). The record layout of the training DB 51 is the same as the table illustrating the sample compositions described using FIG. 3, and content of each component is recorded for each lot. Illustration of the record layout of the training DB 51 is omitted.

FIG. 9 is an explanatory diagram illustrating a configuration of an information processing apparatus 10 used to generate the model 56. The information processing apparatus 10 includes a control unit 11, a main storage device 12, an auxiliary storage device 13, a communication unit 14, an output unit 15, an input unit 16, a reading unit 19, and a bus.

The control unit 11 is an arithmetic and control device that executes a program according to the present embodiment. As the control unit 11, one or more CPUs (Central Processing Units), GPUs (Graphics Processing Units), TPUs (Tensor Processing Units), multi-core CPUs and the like are used. The control unit 11 is connected to each hardware component included in the information processing apparatus 10 via the bus.

The main storage device 12 is a storage device such as an SRAM (Static Random Access Memory), a DRAM (Dynamic Random Access Memory), or a flash memory. The main storage device 12 temporarily stores information required during processing performed by the control unit 11 and programs being executed by the control unit 11.

The auxiliary storage device 13 is a storage device such as an SRAM, a flash memory, a hard disk, or a magnetic tape. The auxiliary storage device 13 stores the training DB 51, the model 56 being generated, programs executed by the control unit 11, and various data required for execution of the programs. The training DB 51 may be stored in an external large-capacity storage device connected to the information processing apparatus 10. The communication unit 14 is an interface that performs communication between the information processing apparatus 10 and a network.

The output unit 15 is, for example, a liquid-crystal display device or an organic EL (Electro Luminescence) display device. The input unit 16 is, for example, an input device such as a keyboard, a mouse, a trackball, or a microphone. The output unit 15 and the input unit 16 may be integrally stacked to constitute a touch panel.

A portable recording medium 96 is, for example, a USB (Universal Serial Bus) memory, a CD-ROM (Compact Disc Read-Only Memory), a magneto-optical disc medium, another optical disc medium, or an SD memory card. The portable recording medium 96 stores a program 97 described later.

The reading unit 19 is an interface capable of reading the portable recording medium 96, such as a USB connector, a CD-ROM drive, or an SD memory reader. The semiconductor memory 98 stores the program 97 and is a memory that may be installed inside the information processing apparatus 10.

The information processing apparatus 10 is a general-purpose personal computer, a tablet, a large-scale computer, a virtual machine operating on the large-scale computer, or a quantum computer. The information processing apparatus 10 may include hardware such as a plurality of personal computers or large-scale computers that perform distributed processing. The information processing apparatus 10 may be configured as a cloud computing system. The information processing apparatus 10 may include hardware such as a plurality of personal computers or large-scale computers that operate in cooperation with each other.

The program 97 is recorded on the portable recording medium 96. The control unit 11 reads the program 97 via the reading unit 19 and stores the program 97 in the auxiliary storage device 13. The control unit 11 may read the program 97 stored in the semiconductor memory 98. Furthermore, the control unit 11 may download the program 97 from another server computer (not illustrated) connected via the communication unit 14 and a network (not illustrated), and store the program 97 in the auxiliary storage device 13.

The program 97 is installed as a control program of the information processing apparatus 10, loaded into the main storage device 12, and executed. The program 97 of the present embodiment is an example of a program product.

The program 97 may be constructed and used on an open software platform. By using an open software platform, the program 97 that is easily portable to various hardware can be realized.

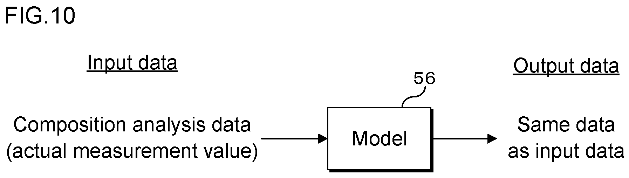

FIG. 10 is an explanatory diagram illustrating the model 56. As described above, the model 56 is a machine-learning model trained to output the same data as the input composition analysis data. Note that, as described using FIG. 3, the composition analysis data is a one-dimensional (1D) matrix in which numerical values indicating content of each component are arranged. That is, the model 56 receives a 1D matrix as input and outputs a 1D matrix of the same size.

The model 56 has, for example, an autoencoder structure including an encoder and a decoder. The model 56 may be generated using an algorithm such as Transformers or LSTM (Long Short-Term Memory).

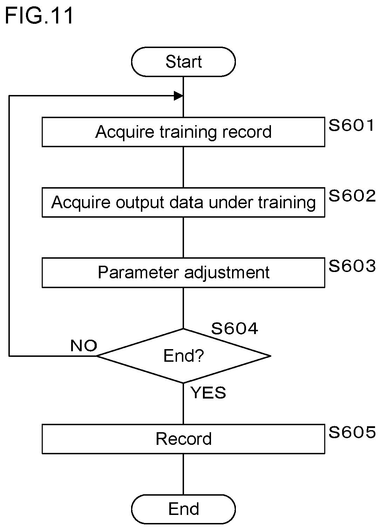

FIG. 11 is a flowchart illustrating a processing flow of a program for generating the model 56. Prior to execution of the program of FIG. 11, for example, an untrained autoencoder model is prepared. By executing the program of FIG. 11, parameters of the untrained model are adjusted, and the model 56 described using FIG. 10 is generated.

The control unit 11 acquires a training record from the training DB 51 (step S601). The control unit 11 inputs composition analysis data included in the acquired training record to the model 56 under training, and acquires an 1D matrix as output (step S602). In the following description, the matrix output from the model 56 under training is referred to as in-training output data.

The control unit 11 adjusts parameters of the model 56 using a method such as backpropagation so that a difference between the composition analysis data input to the model 56 and the in-training output data output from the model 56 is reduced (step S603).

The control unit 11 determines whether or not to end parameter adjustment (step S604). For example, when training using all training data recorded in the training DB 51 has been completed, the control unit 11 determines to end parameter adjustment. When the number of training data items recorded in the training DB 51 is sufficiently large, the control unit 11 may determine to end the process after training has been repeated a predetermined number of times defined by hyperparameters.

When it is determined not to end the process (NO in step S604), the control unit 11 returns to step S601. When it is determined to end the process (YES in step S604), the control unit 11 records the model 56 including the adjusted parameters in the auxiliary storage device 13 (step S605). Thereafter, the control unit 11 ends the process. Thus, generation of the model 56 is completed.

[Verification stage]

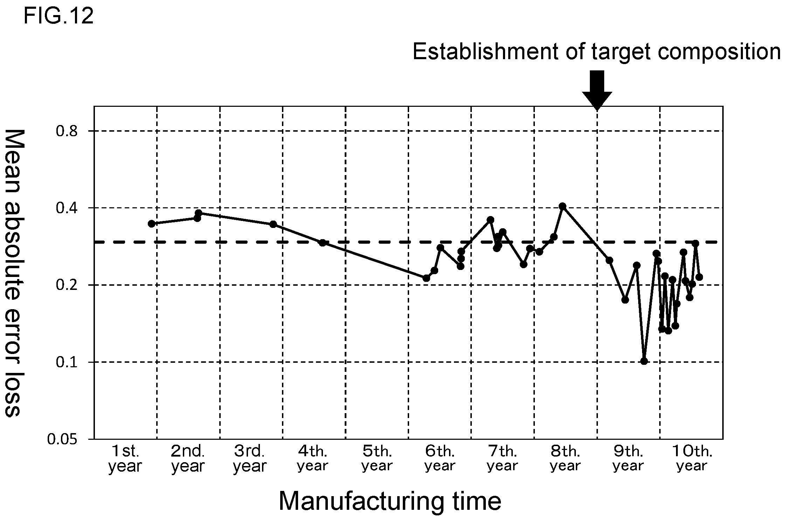

FIG. 12 is a graph illustrating variation in mean absolute error loss according to manufacturing time. A horizontal axis indicates a manufacturing time of steel. A vertical axis indicates mean absolute error loss calculated from the composition analysis data of steel and output data output by the model 56 by inputting the composition analysis data to the model 56. The mean absolute error loss is defined by Equation (1). Based on the target composition described in the data collection stage, 18 lots of steel in which chromium and vanadium are increased within a standard range are manufactured (18 black circles, after the time of target composition establishment).
$$\text{Mean Absolute Error Loss} = \frac{1}{N} \sum_{i=1}^{N} |Y_i - X_i| \quad \cdots\cdots \quad (1)$$

N is the number of components included in the composition analysis data.
$X_i$ is the content of the ith component in the composition analysis data.
$Y_i$ is the ith data of the matrix output from the model 56.

FIG. 12 is a graph plotting mean absolute error losses calculated by inputting composition analysis data of each lot of steel into the completed model 56. A horizontal axis indicates the time of manufacture of the steel. Scale marks on the horizontal axis indicate the number of years since the start of steel manufacturing according to the present embodiment. Each scale line indicates January 1 of each year. A vertical axis indicates the mean absolute error loss. Each black circle indicates data related to one lot of steel.

As illustrated in FIG. 12, steel used in the present embodiment has been manufactured since the end of the first year, and production frequency increased from the first half of the sixth year. From the end of the first year to the middle of the eighth year, manufacturing was performed based on the same standard. In the latter half of the eighth year, the preliminary study stage was executed and the target composition was established. Samples used in the preliminary study stage are not plotted in FIG. 12.

From the beginning of the ninth year, the data collection stage was started, and manufacturing based on manufacturing conditions modified according to the target composition was performed. Samples in the data collection stage are plotted in FIG. 12. Composition analysis data of steel manufactured in accordance with the target standard from the beginning of the ninth year to around autumn of the tenth year were recorded in the training DB 51 and used for generation of the model 56.

When comparing steel of lots manufactured before establishment of the target composition with steel of lots manufactured after establishment of the target composition, it is understood that mean absolute error loss is clearly lower for steel of lots manufactured after establishment of the target composition.

A broken line indicates a reference value of mean absolute error loss determined by experts such as manufacturing engineers based on the data plotted in FIG. 12. In FIG. 12, the reference value is set to a value slightly larger than a maximum value of the mean absolute error loss after establishment of the target composition. That is, the reference value is set to a value smaller than a maximum value of the mean absolute error loss before establishment of the target composition.

[Utilization stage]

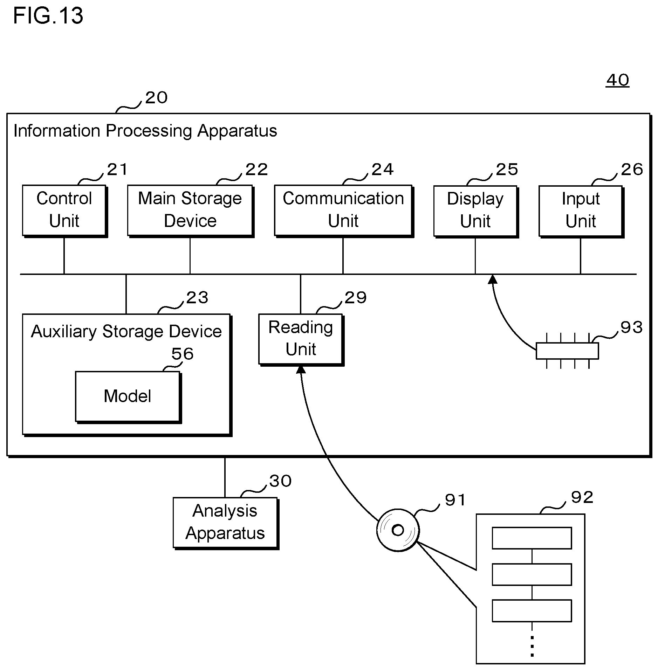

FIG. 13 is an explanatory diagram illustrating a configuration of a steel evaluation system 40. The steel evaluation system 40 includes an information processing apparatus 20 and an analysis apparatus 30. The analysis apparatus 30 is, for example, an ICP-OES (Inductively Coupled Plasma Optical Emission Spectrometer), and is used for composition analysis of steel.

The information processing apparatus 20 includes a control unit 21, a main storage device 22, an auxiliary storage device 23, a communication unit 24, an output unit 25, an input unit 26, a reading unit 29, and a bus.

The control unit 21 is an arithmetic and control device that executes a program according to the present embodiment. One or more CPUs, GPUs, or multi-core CPUs and the like are used as the control unit 21. The control unit 21 is connected to each hardware component included in the information processing apparatus 20 via the bus.

The main storage device 22 is a storage device such as an SRAM, a DRAM, or a flash memory. The main storage device 22 temporarily stores information required during processing performed by the control unit 21 and programs being executed by the control unit 21.

The auxiliary storage device 23 is a storage device such as an SRAM, a flash memory, a hard disk, or a magnetic tape. The auxiliary storage device 23 stores the model 56, programs executed by the control unit 21, and various data required for execution of the programs. The communication unit 24 is an interface that performs communication between the information processing apparatus 20 and a network.

The output unit 25 is, for example, a liquid-crystal display device or an organic EL display device. The input unit 26 is, for example, an input device such as a keyboard, a mouse, a trackball, or a microphone. The output unit 25 and the input unit 26 may be integrally stacked to constitute a touch panel.

A portable recording medium 91 is, for example, a USB memory, a CD-ROM, a magneto-optical disc medium, another optical disc medium, or an SD memory card. The portable recording medium 91 stores a program 92 described later.

The reading unit 29 is, for example, an interface capable of reading the portable recording medium 91, such as a USB connector, a CD-ROM drive, or an SD memory reader. A semiconductor memory 93 stores the program 92 and is a memory that may be installed inside the information processing apparatus 20.

The information processing apparatus 20 is a general-purpose personal computer, a tablet, a large-scale computer, a virtual machine operating on a large-scale computer, or a quantum computer. The information processing apparatus 20 may include hardware such as a plurality of personal computers or large-scale computers that perform distributed processing. The information processing apparatus 20 may include a cloud computing system. The information processing apparatus 20 may include hardware such as a plurality of personal computers or large-scale computers that operate in cooperation with each other.

The information processing apparatus 20 may be configured integrally with the analysis apparatus 30 and may also serve as a control device of the analysis apparatus 30. The analysis apparatus 30 may be configured to automatically transmit composition analysis data to the information processing apparatus 20. A user may read composition analysis data recorded in the analysis apparatus 30 in the information processing apparatus 20 via, for example, a network drive or an arbitrary recording medium.

The program 92 is recorded on the portable recording medium 91. The control unit 21 reads the program 92 via the reading unit 29 and stores the program in the auxiliary storage device 23. In addition, the control unit 21 may read the program 92 stored in the semiconductor memory 93. Furthermore, the control unit 21 may download the program 92 from another server computer (not illustrated) connected via the communication unit 24 and a network (not illustrated), and store the program 92 in the auxiliary storage device 23.

The program 92 is installed as a control program of the information processing apparatus 20, and is loaded into the main storage device 22 and executed. The program 92 according to the present embodiment is an example of a program product.

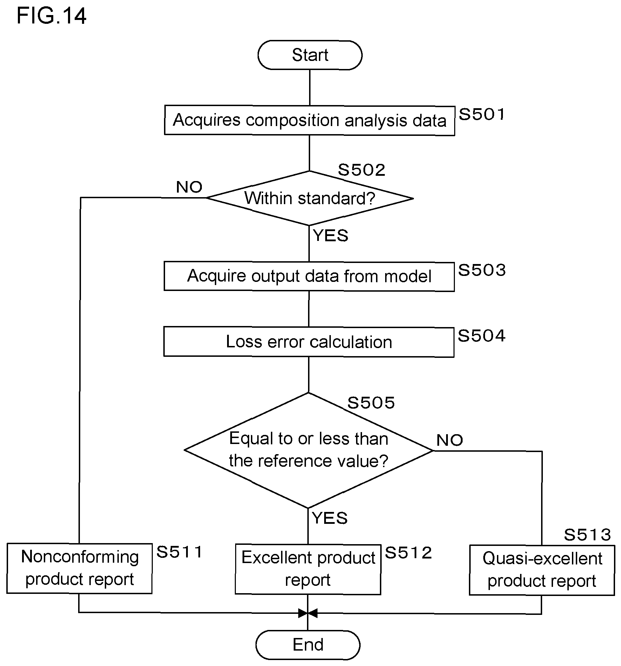

FIG. 14 is a flowchart illustrating a processing flow of the program in the utilization stage. The control unit 21 acquires composition analysis data from the analysis apparatus 30, a network drive or the like (step S501). The control unit 21 determines whether or not the content of each component is within a range defined by the standard (step S502).

When it is determined that there is a component not within the range defined by the standard (NO in step S502), the control unit 21 reports that steel related to the composition analysis data acquired in step S501 is a nonconforming product (step S511). The report is, for example, transmitted to an automatic machine, and the automatic machine marks target steel to indicate that the target steel cannot be shipped. The report may be transmitted to a manufacturing technology engineer.

When it is determined that all components are within the range defined by the standard (YES in step S502), the control unit 21 inputs the composition analysis data acquired in step S501 to the model 56 and acquires output data from the model 56 (step S503). The control unit 21 calculates error loss between the composition analysis data and the output data based on, for example, Equation (1) (step S504).

The control unit 21 determines whether or not the calculated error loss is less than or equal to the reference value (step S505). When the error loss is determined to be less than or equal to the reference value (YES in step S505), the control unit 21 reports that the steel related to the composition analysis data acquired in step S501 is an excellent product (step S512). The report is, for example, transmitted to the automatic machine, and the automatic machine marks target steel to indicate that the target steel is an excellent product. The report may be transmitted to the manufacturing technology engineer.

When the calculated error loss is determined to be larger than the reference value (NO in step S505), the control unit 21 reports that the steel related to the composition analysis data acquired in step S501 is a quasi-excellent product that is neither an excellent product nor a nonconforming product (step S513). The report is, for example, transmitted to the manufacturing technology engineer. The manufacturing technology engineer performs various additional inspections described with reference to FIG. 2.

After completion of step S511, step S512, or step S513, the control unit 21 ends the process.

According to the present embodiment, it is possible to provide a steel evaluation method capable of evaluating, in more detail, steel which is determined to be a conforming product by an existing inspection method. Among steel that is a conforming products satisfying the standard, excellent products having particularly favorable properties and other quasi-excellent products can be easily distinguished. Therefore, for example, excellent products can be preferentially delivered to customers that require particularly high reliability.

According to the present embodiment, by establishing the target composition, the frequency of occurrence of excellent products can be increased. Therefore, an overall improvement in steel quality can be realized.

According to the present embodiment, by utilizing composition analysis data of steel manufactured before establishment of the target composition in establishing the reference value, it is unnecessary to manufacture and test samples for comparative evaluation after completion of the data collection stage, and the reference value can be promptly determined.

According to the present embodiment, by using the reference value, deviations in composition during the manufacturing process can be promptly detected, and to manufacture steel having stable quality.

As the error loss, a loss mean squared error (LOSS MSE) may be used. The LOSS MSE is defined by Equation (2).

$$\text{LOSS MSE} = \frac{1}{N} \sum_{i=1}^{N} \left( Y_i - X_i \right)^2 \quad \cdots \cdots \quad (2)$$

N is the number of components included in the composition analysis data.

$X_i$ is content of an ith component in the composition analysis data.

$Y_i$ is an ith element of the matrix output from the model 56.

Even when the error loss is defined as the loss mean squared error, if the composition of the input data is similar to the training data used to generate the model 56, the error loss becomes small. Conversely, when a difference between the composition of the input data and the training data is large, the error loss becomes large.

As other error losses, any loss function used for generation and evaluation of a machine learning model may be used, such as the square root of the LOSS MSE described above, a loss mean squared logarithmic error (LOSS MSLE), the square root of the LOSS MSLE, Huber loss, Poisson loss, hinge loss, or Kullback-Leibler divergence (KLD).

Note that the loss function used when adjusting parameters of the model 56 by machine learning with the training DB 51 (FIG. 11, step S603) and the error loss used when establishing the reference value in the verification stage may be the same function or different functions. The same function is used as the error loss used when establishing the reference and the error loss calculated in the utilization stage (FIG. 14, step S504).

A computer program can be deployed on a single computer or at one site, or distributed over a plurality of sites and executed on a plurality of computers interconnected via a communication network.

Technical features (constituent elements) described in the respective embodiments can be combined with each other, and new technical features can be formed by such combinations.

The embodiments disclosed herein are to be considered illustrative in all respects and not restrictive. The scope of the invention is indicated by the claims rather than by the foregoing description, and all changes that come within the meaning and range equivalent to the claims are intended to be encompassed.

The independent claims and dependent claims described in the claims can be combined with each other in any and all combinations regardless of the citation format. Furthermore, the claims use a format in which a claim cites two or more other claims (multi-claim format). However, the invention is not limited thereto. A multi-claim that cites at least one multi-claim (multi-multi claim) may be used. Reference Signs List

10　　information processing apparatus

| 11 | control unit |
| 12 | main storage device |
| 13 | auxiliary storage device |
| 14 | communication unit |
| 15 | output unit |
| 16 | input unit |
| 19 | reading unit |
| 20 | information processing apparatus |
| 21 | control unit |
| 22 | main storage device |
| 23 | auxiliary storage device |
| 24 | communication unit |
| 25 | output unit |
| 26 | input unit |
| 29 | reading unit |
| 30 | analysis apparatus |
| 40 | steel evaluation system |
| 51 | training DB |
| 56 | model |
| 91 | portable recording medium |
| 92 | program |
| 93 | semiconductor memory |
| 96 | portable recording medium |
| 97 | program |
| 98 | semiconductor memory |

**Claims**

1. A method for evaluating steel, the method comprising:

   analyzing a composition of steel;
   when composition data obtained by analysis satisfies a predetermined standard, inputting the composition data to a model trained by machine learning to output the same data as input data, and calculating an error loss between output data from the model and the composition data; and
   determining whether or not the error loss exceeds a predetermined reference value.

2. The method according to claim 1, wherein when it is determined that the error loss exceeds the predetermined reference value, an additional inspection is performed on the steel.

3. The method according to claim 1, wherein the model is an autoencoder model including an encoder and a decoder.

4. The method according to any one of claims 1 to 3, wherein:

   the model is trained by machine learning using, as training data, composition data of steel manufactured after a manufacturing process is changed, and
   the reference value is set to a value that is larger than a maximum value of error loss between output data obtained by inputting composition data included in the training data into the model after completion of machine learning and the composition data, and smaller than a maximum value of error loss between output data obtained by inputting composition data of steel manufactured before the manufacturing process is changed into the model after completion of machine learning and the composition data.

5. A program causing a computer to execute processing of:

   acquiring composition data of steel;
   when the composition data satisfies a predetermined standard, inputting the composition data to a model trained by machine learning to output the same data as input data, and calculating an error loss between output data from the model and the composition data; and
   determining whether or not the error loss exceeds a predetermined reference value.

**6.** A method of generating a model, the method comprising:

acquiring training data in which a plurality of sets of composition data indicating contents of respective components of steel are recorded; and

generating, by machine learning based on the acquired training data, a model configured to output the same data as input composition data when the composition data is input.

FIG.1

Preliminary study stage

| Prepare samples differing in composition | → | Composition analysis | → | Property evaluation |

**Establishment of target composition**

Data collection stage

| Manufacturing | → | Composition analysis |
| Manufacturing | → | Composition analysis |
| Manufacturing | → | Composition analysis |

Training DB creation 51

Model generation

Verification stage

| Composition analysis data (actual measurement value) | → | Model 56 | → | Output data |

Loss error calculation

**Establishment of Reference value**

Utilization stage

Utilize for evaluation and classification of product

# FIG.2

Utilization stage

## FIG.3

| Sample no. | Component [ Mass %] | | | | | | | | | | | Remainder |
|---|---|---|---|---|---|---|---|---|---|---|---|---|
| | C | Si | Mn | P | S | Ni | Cr | W | Mo | V | Al | |
| Std | 0.64 | 1.52 | 0.84 | 0.02 | 0.05 | 0.07 | 4.79 | 0.02 | 1.45 | 0.11 | 0.07 | Fe and inevitable impurities |
| MoL | 0.63 | 1.50 | 0.83 | 0.02 | 0.06 | 0.07 | 4.77 | 0.03 | 1.19 | 0.09 | 0.07 | |
| MoH | 0.64 | 1.50 | 0.83 | 0.02 | 0.06 | 0.07 | 4.75 | 0.03 | 1.78 | 0.10 | 0.08 | |
| CrL | 0.64 | 1.50 | 0.83 | 0.02 | 0.06 | 0.07 | 3.99 | 0.03 | 1.48 | 0.10 | 0.07 | |
| CrH | 0.64 | 1.49 | 0.84 | 0.02 | 0.06 | 0.07 | 5.92 | 0.03 | 1.49 | 0.10 | 0.08 | |
| VH | 0.63 | 1.50 | 0.82 | 0.02 | 0.06 | 0.07 | 4.75 | 0.03 | 1.48 | 0.29 | 0.07 | |
| SiL | 0.64 | 1.00 | 0.82 | 0.02 | 0.05 | 0.07 | 4.74 | 0.03 | 1.50 | 0.09 | 0.07 | |
| MnH | 0.64 | 1.51 | 1.16 | 0.02 | 0.05 | 0.07 | 4.74 | 0.03 | 1.49 | 0.09 | 0.08 | |

FIG.4

FIG.5

(a) CrL

(b) Std

(c) CrH

Heat-treatment-induced dimensional-change rate [%]

Tempering temperatures [°C]

CrL_L
CrL_W
CrL_T
ave±3s

Std_L
Std_W
Std_T
ave±3s

CrH_L
CrH_W
CrH_T
ave±3s

FIG.6

(a)

Std

(b)

VH

EP 4 786 974 A1

FIG.7

（a）

SiL

（b）

Std

FIG.8

（a）

Std

（b）

MnH

EP 4 786 974 A1

FIG.9

FIG.10

Input data                                                    Output data

                                                        56

Composition analysis data        ┌──────────┐      Same data
(actual measurement value)  ───▶  │  Model   │  ───▶  as input data
                                   └──────────┘

FIG.11

```
            ┌─────────┐
            │  Start  │
            └─────────┘
                 │
    ┌──────────────────────────────┐
    │     Acquire training record   │  S601
    └──────────────────────────────┘
                 │
    ┌──────────────────────────────┐
    │ Acquire output data under training │  S602
    └──────────────────────────────┘
                 │
    ┌──────────────────────────────┐
    │     Parameter adjustment      │  S603
    └──────────────────────────────┘
                 │
              S604
      NO  ◇  End?  ◇
                 │ YES
    ┌──────────────────────────────┐
    │            Record             │  S605
    └──────────────────────────────┘
                 │
            ┌─────────┐
            │   End   │
            └─────────┘
```

FIG.12

FIG.13

FIG.14

```
                    ┌─────────────┐
                    │    Start    │
                    └──────┬──────┘
                           │
        ┌──────────────────┴──────────────────┐
        │  Acquires composition analysis data  │ S501
        └──────────────────┬──────────────────┘
                           │
                         S502
   NO              ╱─────────────╲
 ┌─────────────── ◁  Within standard?  ▷
 │                 ╲─────────────╱
 │                        │ YES
 │         ┌──────────────┴──────────────┐
 │         │  Acquire output data from model  │ S503
 │         └──────────────┬──────────────┘
 │                        │
 │         ┌──────────────┴──────────────┐
 │         │     Loss error calculation     │ S504
 │         └──────────────┬──────────────┘
 │                        │
 │                      S505
 │            ╱─────────────────╲
 │           ╱ Equal to or less than ╲   NO
 │          ◁  the reference value?  ▷───────┐
 │           ╲─────────────────╱             │
 │                   │ YES                    │
 │                   │                        │
 │                S513                        │
┌──────────┐  S511  ┌──────────┐  S512  ┌──────────────┐
│Nonconforming│    │Excellent product│   │ Quasi-excellent │
│product report│    │    report    │    │ product report │
└─────┬────┘       └─────┬────┘        └───────┬──────┘
      │                  │                     │
      └──────────────────┼─────────────────────┘
                         │
                    ┌────┴────┐
                    │   End   │
                    └─────────┘
```

## INTERNATIONAL SEARCH REPORT

| International application No. |
| --- |
| **PCT/JP2024/033968** |

**A.  CLASSIFICATION OF SUBJECT MATTER**

*G01N 33/2028*(2019.01)i; *C22C 38/00*(2006.01)i; *C22C 38/60*(2006.01)i; *G06N 3/0455*(2023.01)i; *G06N 20/00*(2019.01)i
FI:  G01N33/2028; G06N3/0455; G06N20/00; C22C38/00 301Z; C22C38/60

According to International Patent Classification (IPC) or to both national classification and IPC

**B.  FIELDS SEARCHED**

Minimum documentation searched (classification system followed by classification symbols)

G01N33/2028; C22C38/00; C22C38/60; G06N3/0455; G06N20/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); Scopus

**C.  DOCUMENTS CONSIDERED TO BE RELEVANT**

| Category* | Citation of document, with indication, where appropriate, of the relevant passages | Relevant to claim No. |
| --- | --- | --- |
| Y | WO 2017/200096 A1 (NIPPON STEEL & SUMITOMO METAL CORPORATION) 23 November 2017 (2017-11-23) paragraphs [0003], [0022], [0028]-[0030], fig. 1 | 1-3, 5-6 |
| A | entire text, all drawings | 4 |
| Y | JP 2020-47010 A (DENSO IT LABORATORY INC.) 26 March 2020 (2020-03-26) paragraphs [0003]-[0007], [0101] | 1-3, 5-6 |
| Y | JP 2020-61042 A (AISIN AW CO., LTD.) 16 April 2020 (2020-04-16) paragraphs [0016], [0019]-[0021] | 1-3, 5-6 |
| Y | JP 2019-49778 A (NIPPON TELEGRAPH AND TELEPHONE CORPORATION) 28 March 2019 (2019-03-28) paragraphs [0002], [0006] | 1-3, 5-6 |
| A | WO 2022/168167 A1 (NIPPON STEEL CORPORATION) 11 August 2022 (2022-08-11) paragraphs [0020], [0030], [0054]-[0055] | 1-6 |

☑ Further documents are listed in the continuation of Box C.      ☑ See patent family annex.

| | |
| --- | --- |
| *  Special categories of cited documents: | "T"  later document published after the international filing date or priority date and not in conflict with the application but cited to understand the principle or theory underlying the invention |
| "A"  document defining the general state of the art which is not considered to be of particular relevance | |
| "D"  document cited by the applicant in the international application | "X"  document of particular relevance; the claimed invention cannot be considered novel or cannot be considered to involve an inventive step when the document is taken alone |
| "E"  earlier application or patent but published on or after the international filing date | |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **07 November 2024** | **19 November 2024** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**

| International application No. |
|---|
| **PCT/JP2024/033968** |

**C. DOCUMENTS CONSIDERED TO BE RELEVANT**

| Category* | Citation of document, with indication, where appropriate, of the relevant passages | Relevant to claim No. |
|---|---|---|
| A | 青柳里果, 外, 鉄鋼試料中水素拡散評価を目指したマルチモーダルデータ解析, 表面と真空, 2021, vol. 64, no. 10, pp. 472-475, doi: 10.1380/vss.64.472, (AOYAGI, Satoka et al., Multimodal Data Analysis for Evaluating Hydrogen Diffusion in Steel, Vacuum and Surface Science)<br>p. 472, right column, 2nd paragraph to p. 473, left column, 1st paragraph, p. 473, left column, 6th paragraph, p. 474, right column, 6th paragraph to p. 475, left column, 1st paragraph, fig. 5 | 1-6 |
| A | YOUKACHEN, S. et al. Defect Segmentation of Hot-rolled Steel Strip Surface by using Convolutional Auto-Encoder and Conventional Image processing. 2019 10th International Conference of Information and Communication Technology for Embedded Systems (IC-ICTES). Bangkok, Thailand. 2019, 2019 10th, pp. 1-5, doi: 10.1109/ICTEmSys.2019.8695928<br>entire text, all drawings | 1-6 |

Form PCT/ISA/210 (second sheet) (July 2022)

## INTERNATIONAL SEARCH REPORT
### Information on patent family members

International application No.

**PCT/JP2024/033968**

| Patent document cited in search report | | | Publication date (day/month/year) | Patent family member(s) | Publication date (day/month/year) |
|---|---|---|---|---|---|
| WO | 2017/200096 | A1 | 23 November 2017 | US 2019/0161839 A1 paragraphs [0004], [0023], [0046]-[0083], fig. 1 | |
| JP | 2020-47010 | A | 26 March 2020 | (Family: none) | |
| JP | 2020-61042 | A | 16 April 2020 | (Family: none) | |
| JP | 2019-49778 | A | 28 March 2019 | (Family: none) | |
| WO | 2022/168167 | A1 | 11 August 2022 | US 2024/0018617 A1 paragraphs [0081], [0095], [0119]-[0120] EP 4289988 A1 CN 116601316 A KR 10-2023-0126728 A | |

Form PCT/ISA/210 (patent family annex) (July 2022)

**EP 4 786 974 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006152356 A